(19) **Europäisches Patentamt**

**European Patent Office**

**Office européen des brevets**

(11) **EP 0 847 777 B1**

(12) **FASCICULE DE BREVET EUROPEEN**

(45) Date de publication et mention
de la délivrance du brevet:
**26.05.2004 Bulletin 2004/22**

(51) Int Cl.7: **A61N 1/365**

(21) Numéro de dépôt: **97402800.3**

(22) Date de dépôt: **21.11.1997**

(54) **Dispositif médical implantable actif, notamment stimulateur cardiaque, asservi à un signal d'accélération**

Aktive implantierbare medizinische Vorrichtung, insbesondere ein von einem Beschleunigungssignal geregelter Herzschrittmacher

Active implantable medical device, in particular pacemaker controlled by an acceleration signal

(84) Etats contractants désignés:
**BE CH DE FR GB IT LI SE**

(30) Priorité: **21.11.1996 FR 9614164**

(43) Date de publication de la demande:
**17.06.1998 Bulletin 1998/25**

(73) Titulaire: **ELA MEDICAL (Société anonyme)**
**92541 Montrouge (FR)**

(72) Inventeur: **Legay, Thierry**
**91640 - Fontenay Les Briis (FR)**

(74) Mandataire: **Dupuis-Latour, Dominique et al**
**SEP Pagenberg & Associés**
**14 boulevard Malesherbes**
**75008 Paris (FR)**

(56) Documents cités:
**EP-A- 0 259 658        WO-A-88/09684**
**WO-A-96/30080          US-A- 5 031 614**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne les "dispositifs médicaux implantables actifs" tels que définis par la directive 90/385/CEE du 20 juin 1990 du Conseil des communautés européennes., notamment les stimulateurs cardiaques ou défibrillateurs, dont le fonctionnement est asservi à un paramètre recueilli à l'aide d'un capteur.

À cet égard, bien que dans la suite de la description on fasse principalement référence au cas d'un stimulateur cardiaque, l'invention est applicable de façon générale à une très grande variété de dispositifs électroniques.

De tels appareils sont connus pour adapter leurs actions à la valeur mesurée ou calculée d'un paramètre représentatif des besoins métaboliques du porteur de l'appareil.

Dans les cas des stimulateurs cardiaques, ces systèmes concourent à augmenter la fréquence des impulsions de stimulation lorsque l'on détecte une activité croissante du patient porteur de l'appareil, et à diminuer cette fréquence jusqu'à une valeur plancher en cas de diminution de cette activité, tout particulièrement durant les phases de repos du patient.

Le EP-A-0 550 293 (ELA Médical) décrit un tel appareil, où une fréquence de stimulateur cardiaque est asservie sur un paramètre calculé à partir de la mesure d'une accélération au niveau du tronc du patient. L'hypothèse étant que le module de l'accélération au niveau du tronc du patient est lié à l'activité de celui-ci, on augmente la fréquence des impulsions de stimulation lorsque l'on détecte une activité croissante du patient, et on diminue cette fréquence jusqu'à une valeur plancher en cas de diminution de cette activité, tout particulièrement durant les phases de repos du patient.

Plus précisément, le capteur d'activité du patient comporte un, deux ou trois accéléromètres dont les axes sont orthogonaux entre eux (capteur appelé respectivement "1D", "2D" où "3D").

Dans le cas d'un capteur 1D (accéléromètre unidirectionnel), l'accéléromètre est orienté selon un axe antéro-postérieur, c'est-à-dire perpendiculaire à la poitrine. Dans le cas d'un capteur 2D ou 3D, les accéléromètres sont disposés dans le boîtier de façon à ce qu'un axe (ou deux des axes selon le cas) soit situé dans le plan correspondant au grand côté plat du boîtier du stimulateur. Le stimulateur est implanté de telle façon que son axe vertical soit sensiblement parallèle à l'axe vertical du porteur.

Toutefois, le boîtier peut tourner ou se déplacer légèrement dans le corps du patient par rapport à sa position initiale, si bien que les axes des capteurs peuvent se trouver décalés par rapport au repère de référence prédéfini par l'axe vertical du porteur, l'axe latéral (correspondant sensiblement à l'axe des épaules) et l'axe antéro-postérieur (perpendiculaire aux deux précédents et sensiblement perpendiculaire au tronc du patient).

Pour s'affranchir de ces variations et déplacements des axes, c'est-à-dire du déplacement du repère du capteur par rapport au repère de référence du patient, le EP-A-0 550 293 précité propose un stimulateur de type 2D ou 3D dans lequel la fréquence de stimulation est asservie au module des signaux d'accélération captés sur chacun des trois axes. Ce "module" est défini comme la racine carrée de la somme des carrés des amplitudes instantanées données par les accéléromètres sur chacun des deux ou trois axes ; ce module est invariant dans un changement de repère orthonormé et, de plus, il est représentatif de l'ensemble des accélérations recueillies par les divers accéléromètres et donc de l'activité du patient.

La pratique et les études cliniques montrent cependant que les signaux recueillis par les accéléromètres ne sont pas tous représentatifs d'un effort créé ou subi par le porteur et que, de ce fait, la simple mesure du module des signaux d'accélération est un paramètre qui n'est pas toujours bien corrélé à l'effort réel fourni par le patient, et donc aux besoins métaboliques de celui-ci.

Ainsi, on s'aperçoit que les chocs des semelles sur le sol produisent un pic d'accélération non représentatif d'un effort.

Pour cette raison, le signal d'accélération le long de l'axe vertical est plus important lors de la descente d'un escalier que lors de la montée, alors que l'effort développé par le patient est, au contraire, plus intense lors de la montée. De même, les activités de course à pied légère, du fait du phénomène de chocs des talons sur le sol, sont perçues par le capteur vertical de façon démesurée par rapport aux activités de marche.

Inversement, le pédalage sur une bicyclette, qui génère peu de vibrations, est mal interprété, l'asservissement sur un paramètre d'accélération donnant une sous-évaluation de l'effort développé par le patient.

L'un des buts de l'invention est d'éviter ce biais dans l'appréciation de l'effort du patient en s'affranchissant notamment des signaux parasites non représentatifs de l'effort pour éliminer l'influence de ces parasites sur l'asservissement.

Essentiellement, la solution de l'invention consiste à appliquer une limitation à l'accroissement du carré du module instantané de l'accélération afin de "filtrer" les pics d'accélération parasites, cette limitation n'étant appliquée que dans le sens de l'accroissement, et non dans le sens de la décroissance.

Plus précisément, le dispositif de l'invention, qui est du type général décrit par le EP-A-0 550 293 précité, c'est-à-dire comportant une chaîne de mesure comprenant au moins un capteur délivrant un signal d'accélération, et des circuits pour délivrer, à partir de ce signal d'accélération, un paramètre d'asservissement d'au moins une fonction du dispositif, notamment d'une fréquence de stimulation cardiaque, est caractérisé en ce qu'il comporte des moyens pour limiter le paramètre d'asservissement dans le sens de l'accroissement par une limitation du carré du module du signal d'accélération.

Les sous-revendications visent des formes de mise en oeuvre préférentielles de cette technique.

On va maintenant décrire un exemple de mise en oeuvre de l'invention, en référence aux dessins annexés.

La figure 1 est un schéma par blocs d'un circuit d'asservissement mettant en oeuvre les enseignements de l'invention.

Les figures 2 et 3 sont des chronogrammes du paramètre d'asservissement dans diverses situations d'effort, respectivement avec un asservissement selon l'art antérieur, sans limitation, et avec un asservissement selon l'invention, avec limitation.

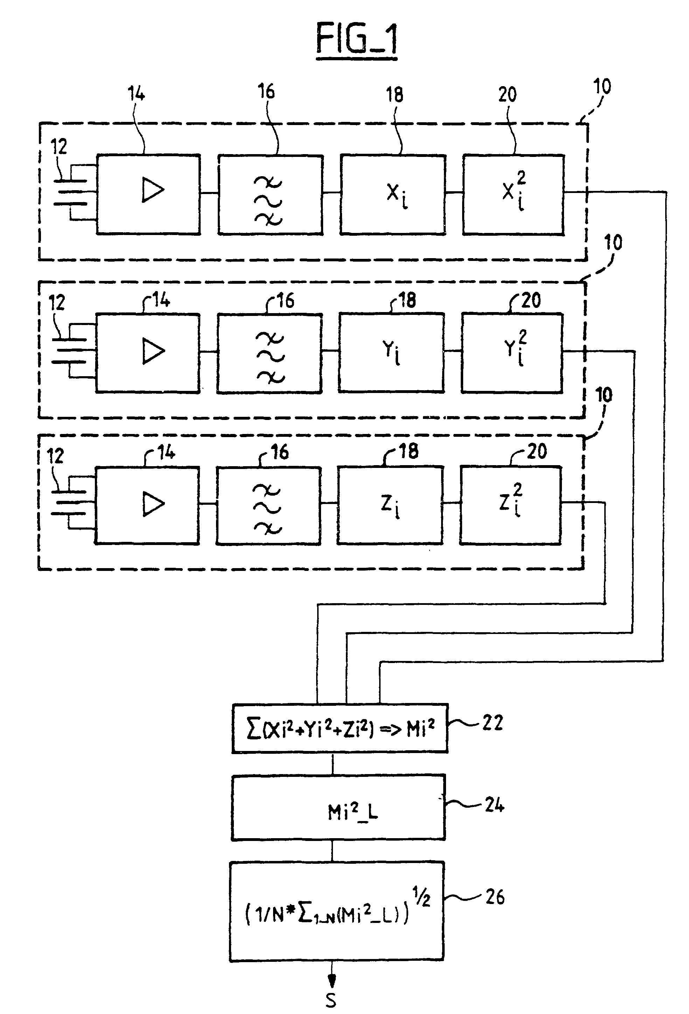

Sur la figure 1, on a représenté un schéma par blocs d'un circuit permettant de produire un paramètre d'asservissement S pour un dispositif implantable actif tel qu'un stimulateur cardiaque.

On décrira ce circuit dans le cas le plus complexe d'un asservissement 3D, impliquant donc trois mesures d'accélération selon trois axes orthogonaux X, Y, Z, mais ce cas n'est pas limitatif ; on peut ainsi appliquer l'invention à un dispositif :

- 1D : l'accéléromètre est disposé suivant l'axe antéro-postérieur ;
- 2D les deux accéléromètres sont disposés suivant l'axe latéral et l'axe vertical et on combine les signaux fournis par ces deux accéléromètres ; ou
- 3D : soit on utilise le module de l'accélération (comme dans l'exemple de la description détaillée qui va suivre), soit, en variante, on opère une combinaison linéaire d'un signal 1D et d'un signal 2D comme ci-dessus.

De la même façon, l'invention sera décrite dans le cas d'un exemple où les signaux sont des signaux échantillonnés numériques, mais cet exemple n'est aucunement limitatif et l'invention est applicable aussi bien à des signaux mesurés et traités par des moyens analogiques.

Le circuit de l'invention comporte tout d'abord trois chaînes de mesure 10 semblables, associées à des capteurs d'accélération respectifs 12 orientés selon trois axes orthogonaux X, Y, Z.

Ces chaînes de mesure sont d'un type en elles-mêmes connu, et l'on pourra se référer à la description détaillée du EP-A-0 550 293 pour de plus amples détails.

Essentiellement, le signal délivré par chacun des accéléromètres 12, qui est par exemple un signal dC de variation de capacité, est appliqué à un étage 14 convertissant les variations de capacité en variations de tension dV et amplifiant ces dernières.

Le signal délivré par cet étage convertisseur et amplificateur 14 est appliqué à un filtre passe-bande 16 pour éliminer, d'une part, les composantes parasites à fréquence élevée (coupure typiquement au-delà de 6 Hz) et, d'autre part, la composante continue du champ de pesanteur terrestre (coupure typiquement en-deçà de 0,6 Hz).

Le signal filtré est ensuite appliqué à un convertisseur analogique-numérique 18 qui échantillonne le signal pour donner une série d'échantillons successifs respectifs $X_i$, $Y_i$ et $Z_i$. Typiquement, le cycle (temps d'intégration) est de 1,5625 s avec une fréquence d'échantillonnage de 20,48 Hz, donnant 32 échantillons par cycle.

Le signal numérique est ensuite appliqué à un étage quadrateur 20, pour donner une valeur respective $X_i^2$, $Y_i^2$ et $Z_i^2$.

Les valeurs $X_i^2$, $Y_i^2$ et $Z_i^2$ sont ensuite sommées par un étage 22 pour donner un module carré instantané $M_i^2 = X_i^2 + Y_i^2 + Z_i^2$.

Le module carré instantané $M_i^2$ est ensuite, de façon caractéristique de l'invention, appliqué à un étage limiteur 24 pour donner un module carré instantané limité, noté $M_i^2\_L$.

Cette limitation (qui peut être interprétée comme un filtrage sélectif, non linéaire) n'est appliquée que dans le sens de l'accroissement du module carré instantané.

Elle peut être mise en oeuvre par l'algorithme d'un logiciel contrôlant un microprocesseur et opérant de la manière suivante.

On définit une pente de limitation SLR (pour *SLew Rate*) à une valeur donnée, par exemple $3906.10^{-6}$ $g^2$ par échelon (g représentant, ici et dans la suite, l'accélération de la pesanteur terrestre), chaque échelon correspondant à un pas d'échantillonnage.

On compare alors le module carré instantané limité de l'échantillon précédent, c'est-à-dire $M_{i-1}^2\_L$, au module carré instantané de l'échantillon actuel, c'est-à-dire $M_i^2$, et on applique la règle suivante :

Si $(M_i^2 - M_{i-1}^2\_L) > SLR$    alors :    $M_i^2\_L = M_{i-1}^2\_L + SLR$

sinon : $M_i^2\_L = M_i^2$

Le module carré instantané limité $M_i^2\_L$ est ensuite appliqué à un étage 26 pour donner le paramètre d'asservissement S, recalculé à chaque cycle, c'est-à-dire toutes les 1,5625 secondes.

Avantageusement, le paramètre d'asservissement est donné par la moyenne quadratique des N échantillons successifs du module carré instantané limité $M_i^2\_L$, c'est-à-dire par la quantité :

$$(1/N^*\Sigma_{1...N}M_i^2\_L)^{1/2}.$$

On notera que, si on travaille sur des signaux analogiques, cette moyenne quadratique correspondra à la valeur efficace du paramètre S(t) sur la durée T de la période d'intégration, à savoir :

$$\left(1/T*\int_T S^2(t)dt\right)^{1/2}.$$

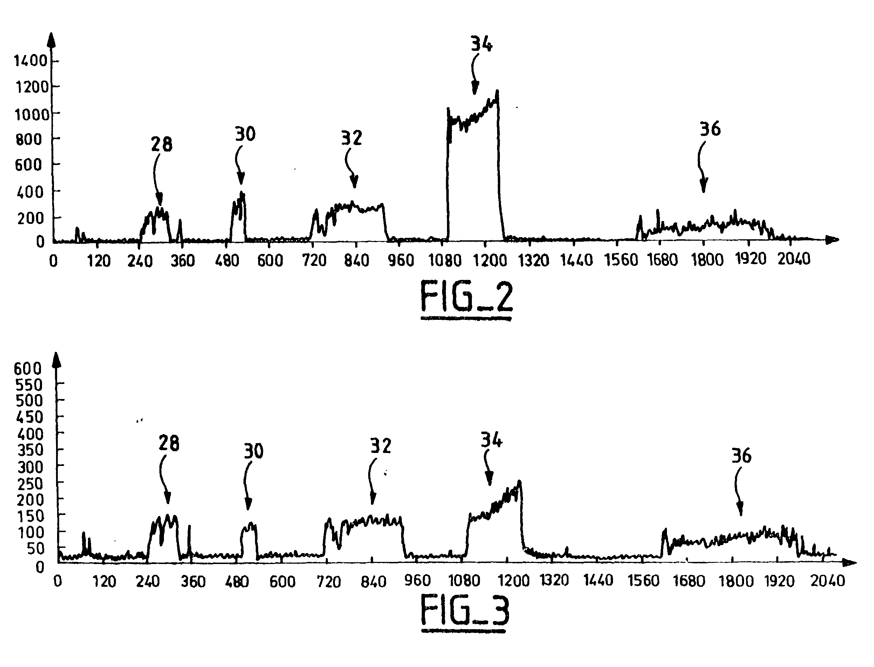

Les figures 2 et 3 illustrent, respectivement dans le cas d'un dispositif de l'art antérieur et dans le cas du dispositif de l'invention, l'évolution du module moyen de l'accélération donnée par un asservissement 2D (c'est-à-dire suivant les axes vertical et latéral) au cours de diverses phases d'activités.

Ces chronogrammes (avec le temps en abscisse) donnent en ordonnée la valeur efficace du module moyen de l'accélération, limitée (figure 3) ou non (figure 2), exprimée en $10^{-6}$ g, g étant la valeur de l'accélération du champ de pesanteur terrestre.

Les phases d'activités reportées sont les suivantes :

- en 28 : montée d'un escalier,
- en 30 : descente d'un escalier,
- en 32 : marche,
- en 34 : course à pied,
- en 36 : pédalage.

Si l'on compare les figures 2 et 3, on voit que la mise en oeuvre de l'étage limiteur de l'invention permet d'obtenir un paramètre d'asservissement plus important pour une montée d'escalier (en 28) que pour une descente d'escalier (en 30) - ce qui est conforme à la réalité physiologique - alors que dans le circuit de l'art antérieur, l'indication était inverse et donc mal corrélée aux besoins métaboliques réels du patient.

De même, en 34, la surévaluation des besoins dans le cas d'une course à pied a été fortement diminuée, et elle est maintenant plus proche de celle de la marche, ce qui est, ici encore, plus physiologique et mieux corrélé à l'évolution requise du rythme cardiaque.

## Revendications

1. Un dispositif médical implantable actif susceptible d'un fonctionnement asservi, notamment un stimulateur cardiaque, ce dispositif comportant une chaîne de mesure (10) comprenant au moins un capteur (12) délivrant un signal d'accélération, et des circuits (22-24) pour délivrer, à partir de ce signal d'accélération, un paramètre (S) d'asservissement d'au moins une fonction du dispositif, notamment d'une fréquence de stimulation cardiaque, **caractérisé en ce qu'**il comporte des moyens (24) pour limiter le paramètre d'asservissement (S) dans le sens de l'acroissement par une limitation du carré du module du signal d'accélération.

2. Le dispositif de la revendication 1, dans lequel la limitation est opérée sur une somme quadratique de composantes d'accélération individuelles ($X_i$, $Y_i$, $Z_i$) selon au moins deux axes distincts délivrées par une pluralité de capteurs respectifs.

3. Le dispositif de la revendication 1, dans lequel toutes les composantes du signal d'accélération sont échantillonnées et appliquées à un étage quadrateur (20) de façon à calculer à chaque instant une valeur ($M_i^2$) liée au module du signal d'accélération.

4. Le dispositif de la revendication 3, dans lequel l'accroissement du paramètre d'asservissement (S) est déterminée par comparaison entre la valeur liée au module du signal d'accélération ($M_i^2$) associée à l'échantilon actuel et la valeur limitée ($M_{i-1}^2\_L$) associée à l'échantillon précédent.

5. Le dispositif de la revendication 4, dans lequel la limitation est opérée en retenant comme valeur limitée ($M_i^2\_L$) soit la valeur associée à l'échantillon actuel ($M_i^2$) si l'accroissement est inférieur à un seuil (SLR) donné, soit la valeur retenue à l'échantillon précédent et augmentée de la valeur du seuil ($M_{i-1}^2\_L + SLR$), dans le cas contraire.

6. Le dispositif de la revendication 1, dans lequel le paramètre d'asservissement (S) est une valeur efficace ou une moyenne quadratique du signal d'accélération limité.

## Patentansprüche

1. Aktive implantierbare medizinische Vorrichtung, zum geregelten Betrieb geeignet, insbesondere ein Herzschrittmacher, wobei die Vorrichtung eine Mess-Kette (10) aufweist, umfassend mindestens einen Sensor (12), welcher ein Beschleunigungssignal liefert und Schaltkreise (22-24) zum Liefern, ausgehend von dem Beschleunigungssignal, eines Regelungsparameters (S) von mindestens einer Funktion der Vorrichtung, insbesondere einer Herz-Stimulationsfrequenz, **dadurch gekennzeichnet, dass** sie Mittel (24) aufweist, zur Begrenzung des Regelungsparameters (S) in der Richtung der Steigerung durch eine Begrenzung des Quadrats des Moduls des Beschleunigungssignals.

2. Vorrichtung gemäß Anspruch 1, in welcher die Begrenzung auf eine quadratische Summe der individuellen Bestandteile der Beschleunigung ($X_i$, $Y_i$, $Z_i$) entlang mindestens zweier unterschiedlicher Achsen ausgeführt wird, welche durch eine Vielzahl von jeweiligen Sensoren geliefert werden.

3. Vorrichtung gemäß Anspruch 1, in welcher alle Bestandteile des Beschleunigungssignals abgetastet und einem Quadrierungsschritt (20) unterworfen werden, um zu jedem Zeitpunkt einen Wert ($M_i^2$) zu berechnen, der zu dem Modul des Beschleunigungssignals gehört.

4. Vorrichtung gemäß Anspruch 3, in welcher die Steigerung des Regelungsparameters (S), welche zu dem Modul des Beschleunigungssignals gehört, durch Vergleich zwischen dem Wert ($M_i^2$) bestimmt wird, der zu dem aktuellen Abtastwert gehört und dem begrenzten Wert ($M_{i-1}^2\_L$), der zu dem vorhergehenden Abtastwert gehört.

5. Vorrichtung gemäß Anspruch 4, in welcher die Begrenzung ausgeführt wird, indem als begrenzter Wert ($M_i^2\_L$) entweder der mit dem aktuellen Abtastwert ($M_i^2$), wenn die Steigerung kleiner ist als ein gegebener Schwellwert (SLR), oder der bei der vorhergehenden Abtastung und mit dem Schwellwert ($M_{i-1}^2\_L + SLR$) erhöhte Wert in dem gegenteiligen Fall, zurückbehalten wird.

6. Vorrichtung gemäß Anspruch 1, in welcher der Regelungsparameter (S) ein Effektivwert oder ein quadratisches Mittel des begrenzten Beschleunigungssignals ist.

**Claims**

1. An active implantable medical device capable of functioning in an enslaved mode, in particular a cardiac pacemaker, said device comprising a measurement unit (10) comprising at least one sensor (12) issuing an acceleration signal, and circuits (22-24) for issuing, from said acceleration signal, a parameter (S) of enslavement of at least one function of the device, in particular a cardiac stimulation frequency, **characterised by** including means (24) for limiting the enslavement parameter (S), in the direction of increase of the same, by a limitation of the square of the module of the acceleration signal.

2. The device of claim 1, wherein the limitation is applied on a quadratic sum of individual acceleration components ($X_i$, $Y_i$, $Z_i$) along at least two different axes, issued by a plurality of respective sensors.

3. The device of claim 1, wherein all the components of the acceleration signal are sampled and applied to a squaring stage (20) in order to compute at each instant a value ($M_i^2$) related to the module of the acceleration signal.

4. The device of claim 3, wherein the increase of the enslavement parameter (S) is determined by a comparison between the value related to the module of the acceleration signal ($M_i^2$) associated to the current sample and the limited value ($M_{i-1}^2\_L$) associated to the previous sample.

5. The device of claim 4, wherein the limitation is applied by retaining as the limited value ($M_{i-1}^2\_L$) either the value associated to the current sample ($M_i^2$) if the increase is less than a given threshold (SLR), or the value retained for the previous sample and increased by the value of the threshold ($M_{i-1}^2\_L+SLR$) in the opposite case.

6. The device of claim 1, in which the enslavement parameter (S) is an efficient value or a quadratic average of the limited acceleration signal.

## FIG_1

$$\sum (Xi^2 + Yi^2 + Zi^2) \Rightarrow Mi^2 \qquad 22$$

$$Mi^2\_L \qquad 24$$

$$\left(1/N^* \sum_{1\_N}(Mi^2\_L)\right)^{1/2} \qquad 26$$

S

FIG_2

FIG_3

EP 0 847 777 B1